Europäisches Patentamt

European Patent Office

Office européen des brevets

(11) Publication number: **0 496 027 A1**

## EUROPEAN PATENT APPLICATION

(21) Application number: **91110171.5**

(22) Date of filing: **20.06.91**

(51) Int. Cl.5: **C12Q 1/66, C12N 15/85,**
**G01N 33/50, C12Q 1/04**

(30) Priority: **25.01.91 IL 97048**

(43) Date of publication of application:
**29.07.92 Bulletin 92/31**

(84) Designated Contracting States:
**AT BE CH DE DK ES FR GB GR IT LI LU NL SE**

(71) Applicant: **R.O.B.I.T. RESEARCH AND**
**DEVELOPMENT COMPANY LTD., c/o EFRATI,**
**GALILI & CO.**
**19 Ahad Haam Str.**
**Tel-Aviv 65541(IL)**

(72) Inventor: **Honigman, Alexander**
**25 Kobobi Str. Ramot Denya**
**Jerusalem 96757(IL)**
Inventor: **Israel, Shoshana**
**8 Keren Kayemet Str.**
**Jerusalem 92465(IL)**

(74) Representative: **Vossius & Partner**
**Siebertstrasse 4 P.O. Box 86 07 67**
**W-8000 München 86(DE)**

(54) **A bioluminescence assay for gene expression in growing mammalian cells.**

(57) A method for detecting the presence of a certain substance in a medium is provided which is based on the use of genetically engineered tester cells which are capable of absorbing and of expressing the luciferase enzyme in the presence of said substance in their surrounding medium. In the performance of this method, cells are incubated with a tested medium and light generation under appropriate conditions is monitored, light generation above background levels being an indication of the presence of said substance in the tested medium.

Fig.4b

The present invention concerns a bioluminescence assay method, kit and system for monitoring the presence in cells or in a medium of certain substances. In accordance with the present invention, genetically engineered cells capable of emitting light when exposed to such substances are utilised.

In the following description, reference will at times be made to various prior art documents by indicating their number from the list of references which is to be found at the end of the description before the claims.

Bioluminescence assays for gene expression, in particular those utilising the firefly enzyme lucerifase, have been described (for review, see **1**). However, when applied to the monitoring of gene expression in living, growing mammalian cells, these assays lead in most cases to the destruction of the cells. In addition, even where cell viability was maintained, such assays proved to be unsensitive.

Accordingly, it was generally believed that bioluminescence cannot be used as an assay system in intact mammalian cells.

It is the object of the present invention to provide a bioluminescence assays for gene expression in living, growing mammalian cells which overcomes the abovementioned deficiencies of the prior art.

More particularly, it is the object of the present invention to provide an assay for detecting the presence of a gene activator substance in a cell, wherein genetically engineered cells capable of light generation in the presence of said substance are used.

It is a further object of the invention to provide a method for detecting the presence of an agent in a medium by utilising genetically engineered cells capable of light generation in the presence of said agent.

It is a further object of a specific embodiment of the present invention to provide bioluminescence assay to determine the occurrence of a viral infection in body fluids of an individual suspected of having such an infection.

It is an object of another specific embodiment of the present invention to provide a method for determining the presence or level in a biological fluid of ligands which bind to receptors on the external cell membrane.

In accordance with the present invention it has surprisingly been found that contrary to prior art general belief, bioluminescence may indeed be used as an assay in growing mammalian cells.

In accordance with the method of the present invention, mammalian cells are genetically engineered with a DNA construct comprising a promoter sequence and a *luc* gene coding for the firefly luciferase. The genetically engineered cells used in accordance with the present invention are cells selected for their capability to absorb luciferin, which is the substrate of luciferase, from a surrounding medium and further for their capability of a high level gene expression, namely high level of production of gene expression products. In the presence of an inducer substance for said promoter in the cell, the luciferase enzyme will be expressed and under appropriate conditions, including the presence of luciferin in the surrounding medium, light will be generated.

The inducer substance which may be detected by the method of the invention may either be one which originates in the external medium, e.g. the *TAX* protein of human T leukemia virus (HTLV) I and II or *TAT* of human immunodeficiency virus (HIV), it may be a substance expressed in the cell, e.g. a viral inducer substance, e.g. TAX or TAT, expressed in the cell after infection thereof by a virus or it may be a second messenger, e.g. a cellular gene product expressed in a cell as a result of a viral activation of a cellular gene or as a result of binding of a ligand to a receptor on the external membrane surface. In each case, as will be readily understood by the artisan, said promoter will be one which is inducible by the respective inducer substance.

Thus, in accordance with the present invention there is provided a method for detecting the presence of an inducer substance in a cell comprising:

a) providing tester cells prepared by

i. constructing an expression vector comprising a promoter inducible by said inducer substance and a a *luc* gene being under the expression control of said promoter, said expression vector being capable of expressing the *luc* gene at a low level in the absence of said inducer substance and at a high level in the presence thereof, and

ii. choosing cells capable of absorbing luciferin from a surrounding medium and of a high level gene expression and transfecting these cells with said expression vector;

b) culturing the tester cells under conditions in which expression of the *luc* gene will result in light generation; and

c) monitoring light generation by appropriate means, light generation above a certain level indicating the presence of said inducer substance in said cell.

The present invention also provides expression vectors and tester cells useful in the above method and processes for their preparation.

It is not entirely clear why the long felt need, which is fulfilled in accordance with the present invention, has not been achieved in accordance with the prior art. It is speculated, that the failure of the prior art investigators resulted from an inappropriate choice of cells and expression vectors. The cells to be used for preparing tester cells of the invention are chosen for their capability to transport luciferin into the cells from a surrounding medium and for their capability of high level gene expression in general and of the reporter gene product, luciferase, in particular. One example of such cells which were utilised in experiments which led to the present invention are cells derived from the 293 cell line. However, it should be noted that other types of cells may also be used for the preparation of tester cells in accordance with the present invention and the artisan will have no difficulties in finding other appropriate cells after a limited amount of experimentation.

Without the following explanation to be considered limiting, it may be that the success in the use of the 293 cell results from the fact that these cells contain the Ela and Elb adenovirus genes the expression product of which are pleotropic inducers, namely inducers which are capable of activating many promoters or increase the inducing activity of specific promoters. It is accordingly believed that other cells containing genes encoding pleotropic inducers, which render them capable of high level gene expression, may also be very suitable for the preparation of tester cells of the invention, e.g. other cells containing the Ela and Elb genes.

The expression vector of the invention is constructed so as to be capable of low expression of the *luc* gene in the absence of said inducer substance and a high level of expression in the presence thereof. It should be noted that many promoters, in particular viral promoters, bring about some expression of their associated gene even in the absence of their inducer. Such background expression will increase the background signal and hence decrease the signal-to-noise ratio in the method of the invention.

The signal-to-noise ratio may be considerably increased by means of promoter occlusion, namely by placing the promoter which is linked to the *luc* gene (to be refered to herein at times as "test promoter") downstream to an occluding promoter, which latter is a promoter which is more active than the test promoter in the absence of the inducer substances of both promoters there being no transcription termination signal between the two promoters. Thus, in the absence of the inducer substance of the test promoter, the expression induced by the occluding promoter will inhibit the expression induced by the test promoter; however, when the inducer substance of the test promoter is present, the activity thereof beyond that of the occluding promoter (hence overcoming the occlussion) and consequently the *luc* gene will be expressed. As a result, the basal light generation will be much lower than that occuring in the presence of said inducer substance which will mean a considerable increase in the signal-to-noise ratio.

By one of its embodiments, the present invention provides a method for the detection of the presence of viable viral particles in a tested sample. For use in such a method, the tester cells have to be capable of being infected by the virus and said promoter in the expression vector should be capable of being induced by a substance encoded by the viral genome. In principle, any virus which genome encodes a recognised inducer substance may be assayed by the method of the present invention and the following are some examples: HIV, the promoter being inducible by HIV *TAT*; HTLV I and II, the promoter being inducible by *TAX* of HTLV I and II, respectively; hepatitis B virus (HBV), the promoter being inducible by the HBV *X* protein; *herpes simplex* virus (HSV), the promoter being inducible by the HSV *ICP4*; cetomegalo virus (CMV), the promoter being inducible by the CMV transactivator; etc.

By another of its embodiments, the present invention provides a method for the detection of the presence of agents in a medium which are capable of being absorbed by the tester cells and either as is or after modification by enzymes present in the cell, serve directly or indirectly as inducers of said promoter. Examples of an agent which can be transported intact across a cell membrane and as is activate a suitable promoter is HIV *TAT*.

By still another of its embodiments, the present invention provides a method for the detection in a medium of substances which are ligands of receptors present on the external membrane surface of the tester cells, the binding of which to the receptors induces the production in the cell of a "second messenger", e.g. transcription factors such as *NFxB* or *SPl*, said promoter in the expression vector being inducible by the second messenger. Examples of such ligands are various hormones, growth factors, etc. For use in accordance with this embodiment, the tester cells should display a receptor for that ligand on their external surface and have a second messenger generating machinery which is activated as a result of binding of said ligand to said receptor. The receptors and said machinery should either be a *priori* present in the parent cell used for preparing tester cells in accordance with the invention, or be introduced into the cell by genetic engineering methods known *per se*.

The present invention further provides a kit and system for carrying out the method of the invention. The kit comprises the ingredients necessary for carrying out the method of the present invention including transfected cells and optionally also appropriate incubation media for the cells, luciferin, buffers for carrying out the light reaction, etc. Such a kit may also comprise various standards, e.g. solutions containing known concentrations of a substance to be tested.

A system for carrying out the method according to the invention will comprise the same ingredients as in the above kits, with the addition of a light monitoring device.

In the following, the present invention will at times be illustrated with reference to the use of tester cells in accordance with the invention for the detection of HIV (AIDS) infections. However, the present invention is not limited thereto, and a person skilled in the art will no doubt appreciate that tester cells transfected with expression vectors comprising promoters other than those described hereinbelow may be prepared. These other promoters would be selected for their specificity to various other inducer substances examples being all the promoters inducible by the viral inducer substances mentioned above.

The expression vector in tester cells useful in the detection of an HIV infection comprise the *luc* gene and an HIV *TAT*-inducible promoter selected from the group consisting of the HIV-LTR or part thereof including the *tar* site, which is the *TAT* protein recognition site, or analogous sequences capable of inducing expression of a downstream DNA sequence in the presence of HIV *TAT*, the *luc* gene being under the expression control of the promoter.

For the generation of mRNA of a gene it is necessary for the expression vector to have an active promoter and a polyadenylating (poly A) site and signal at the 3' end of the gene. Seeing that the poly A site of the *luc* gene is inefficient, it is preferable to include in the expression vector of the present invention, a different poly A signal and site, such as that of the SV40. The expression vector preferably comprises also a marker gene, such as that conferring antibiotic resistance, which facilitates the selection of cells transfected thereby.

As mentioned above, many promoters of viral origin, including the HIV-LTR, are active to some extent even in the absence of their inducer. As also mentioned above, this fact may bring about an increase in the background signal and thereby reduce the signal-to-noise ratio and hence the sensitivity of the assay. This background signal may be reduced to small levels by promoter occlusion, namely by placing the test promoter downstream of an occlusion promoter, which latter promoter is more active than the former one in the absence of the inducer substances of both promoters. An example of an occlusion promoter which may be used for occluding the HIV-LTR is the MLV-LTR. The HIV-LTR directed transcription may either be in the sense orientation relative to the MLV-LTR directed transcription or preferably in the antisense orientation relative thereto. The preference for the antisense orientation is in view of the fact that in such a case the background activity of the test promoter (namely its induced exoression in the absence of its promoter) is reduced even further. When the *TAT* protein is absent, the MLV-LTR, for which there is no inducer substance in the cell, is more active than the former and hence the transcription induced by the HIV-LTR is almost entirely eliminated. This elimination is even stronger when the direction of transcription induced by both promoters is in an antisense orientation relative to one another. When *TAT* is present, the HIV-LTR promoter becomes more active than the MLV-LTR (overcoming the occlusion) and consequently the *luc* gene is expressed.

Tester cells in accordance with the present invention for the detection of an HIV infection are prepared by transfecting the cells with the above expression vector. Such tester cells should detect the presence of the *TAT* protein and/or the viable HIV particles in a tested sample. For such tester cells to be reliable and sensitive detectors of the presence of *TAT* protein they should be capable of absorbing this protein from their surrounding medium. Example of cells which are capable of such absorbtion are cells derived from the 293 cell line.

For assaying HIV infection in the blood, tester cells which have the capability to absorb the *TAT* protein are subjected to a first transactivation stage in which they are brought into contact with a tested blood or other body fluid sample or a fraction thereof which contains the *TAT* protein whenever present in the tested sample. In the blood, the *TAT* protein is found primarily in the nucleus of $T_4$ helper cells and it is accordingly prefered lyse the T4 helper cells prior to carrying out the test. Following such lysis these cells release their content of the *TAT* protein into the medium, and then either the lysate as is or protein-containing fractions thereof are brought into contact with the tester cells. If the tested sample is from an HIV infected individual, *TAT* will be present and its absorption into the tester cells will result in the expression of luciferase.

It is at times preferable to directly assay for the presence of viable viral particles in a body fluid particularly in cases where the tested virus does not induce the production of a diffusible transactivating product. For that purpose, the cell has to be capable of being infected by the tested virus, which usually requires the presence of an appropriate receptor for the virus on the external membrane surface of the cell. Such a viral receptor should +be either a *priori* present in the parent cell from which the tester cells were prepared, or introduced by genetic engineering methods known *per se*. For example, in the case of HIV,

4

direct infection by the virus requires the presence on the external cell membrane of the CD4 receptor and cells chosen for the preparation of the tester cells may be T4 helper cell lines or other cell lines displaying the CD4 receptor, or cells such as 293 transfected with a gene coding for CD4.

The expression of luciferase is assayed by monitoring light emission under appropriate conditions. For such monitoring, the tester cells are incubated in tissue culture medium (e.g. containing 10% calf serum) in the presence of luciferin (e.g. in a final concentration of 0.3 mM) under appropriate conditions (e.g. $CO_2$ - (7%) incubator, 37°C). An appropriate device for monitoring light emission, which has been developed in accordance with the invention, is a camera device which consists of a relatively small light-tight but gas permeable box fitted on top of a photographic film cassette, e.g. a POLAROID (trade mark) film cassette, having a sliding shutter and provided with holding means to hold a carrier such as a dish, slide, plate and the like in contact with the film. In order to monitor light emission, a tester cell-containing carrier is exposed to the film for a sufficient amount of time, depending on the film's sensitivity, the incubation conditions, and the infectivity or titer or the tested virus. Any person skilled in the art will be able to determine with ease the appropriate incubation time in each case. Alternatively, light emission may also be detected in any of a number of available photometers, such as the photo-intensified charge coupled device (CCD). However, it should be pointed out that in addition to the much cheaper and simpler design of the camera device, it also has the advantage that light detection may be carried out within an incubator, which enables a continuous exposure for prolonged periods of time, as well as the continuous monitoring of the same cells at different times.

The method of the present invention proved to be sufficiently sensitive to enable light detection from a single cell. This however usually requires prolonged incubation periods during which light is being monitored, e.g. several hours. To decrease the time required for the detection of the emitted light, the cells may be concentrated e.g. by centrifugation, and light emission may then be measured in this concentrate. By such concentration, all the light generating cells are closely packed and although it does not enable to monitror single cells, the exposure time is considerably reduced, e.g. to several minutes.

It is preferred in accordance with the present invention to compare the results with control results obtained from tester cells incubated under similar conditions with a negative control sample. Increase of light production above control levels would indicate the presence of the tested substance in the sample. Additionally, the amount of emitted light may be compared to the amount of light obtained with various positive control standards.

The present invention will now be illustrated with reference to examples in which tester cells in accordance with the present invention are used to detect the presence of the HIV transactivator protein *TAT*. These examples should be construed as an illustration of the general scope of the invention and should not be considered limiting.

In the following description reference will at times be made to the accompanying drawings, in which:

**Fig. 1** is a schematic representation of the manner of preparation of pALUCAT plasmid which has a *luc* encoding DNA sequence under the expression control of HIV-LTR;

**Fig. 2** is a schematic representation of the manner of preparation of pZipHIVLUC plasmids which have *luc* gene under the expression control HIV-LTR which is downstream to an MLV-LTR;

**Fig. 3** is a schematic representation of the manner of preparation of pZipΔHIVLUC, which are plasmids similar to the plasmids prepared in accordance with Fig. 2 but differ in that the HIV-LTR lacks 160 base pairs;

**Fig. 4** shows in detail the HIV-LTR/luc region of several plasmids used in the experiments below, both plasmids in which the HIV LTR is not occluded (a) and those in which the HIV LTR is occluded by another promoter (b);

**Fig. 5** shows light production by 293 cells transiently transfected with either the plasmid pZipΔHIVLUC4 (a) or pALUCAT (b);

**Fig. 6** shows results of dot-hybridization of a *tat* specific DNA probe to RNA obtained from three clones of 293 cells stably transfected with pRSVtat (3 upper spots), a positive control with the pRSVtat plasmid (lower center spot) and a negative control with RNA obtained from mock transfected 293 cells (lower right corner);

**Fig. 7** shows light production from growing cells of two of the clones in Fig. 6 transfected with the plasmid pZipΔHIVLUC4: a and c - 5 hours of exposure to a light sensitive film; b and d - 17 hours exposure to a light sensitive film;

**Fig. 8** shows light production by 293 growing cells transfected with pZipΔHIVLUC4 exposed to extracts from the three pRSVtat transfected clones and the mock transfected clone of Fig. 6;

**Fig. 9** shows light production by 293 cells stably transfected with pZipΔHIVLUC4 plasmid exposed to extract of either normal 293 cells (a) or extracts of tat transfected 293 cells (b), after an overnight exposure;

**Fig. 10** shows light emission by pelleted 293 cells transfected with pZipΔHIVLUC4 and exposed to extracts of tat transfected cells (exposure time - 2 minutes); and

**Fig. 11** shows light production by growing 293 cells, stably transfected with the plasmid pZipΔHIVLUC4, exposed to various dilutions of extracts of HIV infected cells (HUT-78).

## PREPARATION OF RECOMBINANT DNA MOLECULES

In the following, the preparation of some plasmids is described. DNA cleavage and ligation of the pertinent DNA fragments to construct the plasmids was performed in accordance with methods known *per se*.

At each stage, the constructed plasmids were cloned into E. coli DR 100 cells and the transfected cells were selected for conferred ampicillin resistance by an Amp[R] gene carried by all the plasmids prepared. After selection as specified, plasmids were purified from each of approx. 50 of the selected colonies and restriction analysis of these plasmids was carried out. After such an analysis, one clone was selected, grown, and its plasmids purified, which then served for subsequent stages of plasmid construction or for transfection of tester cells, where appropriate.

### a. Preparation of pALUCAT

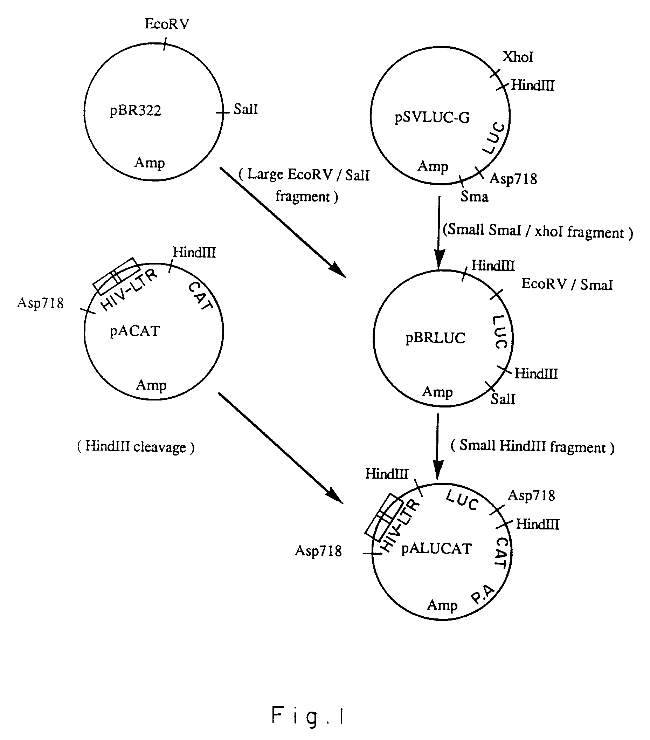

A schematic description of the construction of the plasmid pALUCAT is shown in Fig. 1. The source of the *luc* gene (encoding the enzyme luciferase) was the plasmid pSVLUC-G obtained from J. Collins, FRG. The *luc* gene was isolated and purified following cleavage of pSVLUC-G with SmaI and XhoI restriction enzymes. The plasmid pBR322 (**2**) which carries a gene for ampicillin resistance, was cleaved by SalI and by EcoRV restriction enzymes, the small 466 b.p. fragment discarded, and the purified *luc* fragment from pSVLUC-G was ligated in its place, to give rise to the plasmid pBRLUC.

As illustrated in Fig. 1, the *luc* gene fragment obtained from pSVLUC-G has a single HindIII restriction site close to the XhoI restriction site. The plasmid pBR322 also contains a single HindIII restriction site on the large EcoRV-SalI fragment close to the EcoRV site. Thus, the *luc* gene in pBRLUC is flanked by HindIII restriction sites. pBRLUC was thus cleaved with HindIII and the *luc* gene fragment so obtained was ligated into the HindIII restriction site of pACAT (A. Panet et al., Biotechnology Ceneral-BTG, Nes Ziona, Israel). The plasmid harbours an HIV-LTR fused to and upstream of the *cat* gene.

The resulting pALUCAT plasmid thus comprises the *luc* gene under the expression control of HIV-LTR. This plasmid also comprises the *Amp* gene of pACAT which confers ampicillin resistance.

The pALUCAT plasmid so constructed is suitable for use in transfecting cells to be used as tester cells for detecting the presence of tat in a medium.

The degree of light emission in the pALUCAT carrying tester cells depends on the degree of activation of the HIV-LTR. In order to obtain a proper calibration, cells belonging to the same cell line as the pALUCAT containing cells were transfected by the plasmid pACAT. A calibration is thus obtained by comparing the degree of light emission in the pALUCAT transfected cells to the degree of the CAT production in the pACAT transfected cells.

It should be noted that the HIV-LTR expresses some background level activity even in the absence of the *TAT* protein. Thus, some degree of expression of the *luc* gene occurs continuously even in the absence of the *TAT* protein and therefore, when used in accordance with the method of the invention, cells containing this plasmid show a certain degree of background bioluminescence.

### b. Construction of pZipHIVLUC

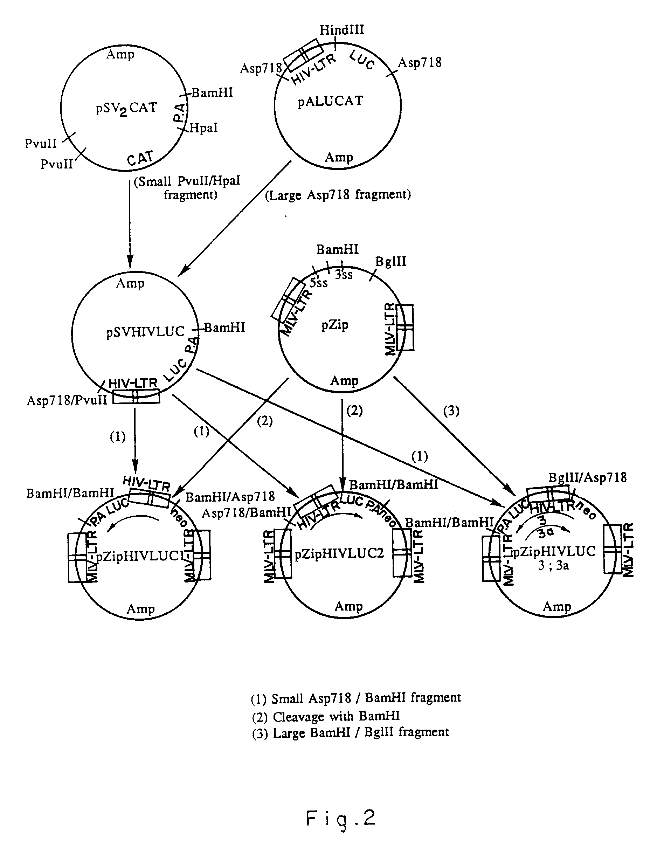

A schematic representation of the construction of the pZipAIVLUC plasmids is shown in Fig. 2.

The pALUCAT plasmid has two Asp718 restriction sites (as shown in Fig. 1) flanking the full HIV-LTR sequence and the *luc* gene. After cleavage of pALUCAT with Asp718, the DNA fragments containing the HIV-LTR and the *luc* gene were isolated and purified and the cohesive ends thereof were filled. The plasmid pSV₂CAT (**3**) was cleaved with PvuII and HpaI restriction enzymes, and the fragment containing the *cat* gene was discarded. The cohesive ends of the remaining part of pSV₂CAT were filled as above, and this fragment was then ligated to the said DNA fragment from pALUCAT. Such a ligation reconstitutes the Asp718 restriction site attached thereto, while the PvuII and the other Asp718 restriction sites are lost.

The resulting pSVHIVLUC plasmid has the HIV-LTR, the *luc* gene and the SV40 polyadenylation signal (P.A. in Fig. 2) between an Asp718 and a BamHI restriction sites.

The plasmid pZip (4) harbours the prokaryotic *bla* gene, which confers ampicillin resistance and an origin of replication of pBR327. The neomycin resistance conferring gene (neo$^R$) and the MLV packaging signal are flanked by MLV-LTR DNA sequences. This plasmid has a unique BamHI restriction site between the donor and acceptor splice sites close to the 5' LTR allowing expression of a cloned gene at the BamHI site, as well as the expression of the neo$^R$ gene.

The DNA fragment located between the Asp718 and BamHI cleavage sites in plasmid pSVHIVLUC, which includes the HIV-LTR and the *luc* gene as well as the poly A signal and site, was isolated. The fragment was ligated to the BamHI cleavage site of pZip, the incompatible cohesive ends were filled in and religated.

Two types of plasmids were produced in this manner, in which the HIV-LTR is occluded by the upstream MLV-LTR:

1) pZipHIVLUC1, in which the HIV-LTR/*luc*/PolyA DNA fragment was inserted in an antisense orientation relative to the MLV-LTR directed transcription, namely in an orientation in which the MLV-LTR directed transcription and the HIV directed transcription are in opposite directions.

2) pZipHIVLUC2, in which the HIV-LTR/*luc*/Poly A fragment was inserted in a sense orientation relative to MLV-LTR directed transcription.

A different set of plasmids was prepared by inserting the HIV-LTR *luc* DNA fragments from pSVHIVLUC between the BamHI and the BglII restriction sites of pZip. Two constructs were thus obtained:

(1) pZipHIVLUC3, in which the orientation of the HIV-LTR/*luc*/PolyA DNA fragment is the same as that of pZipHIVLUC1.

(2) pZipHIVLUC3a, in which the orientation of the HIV-LTR/*luc*/PolyA DNA fragment is the same as that of pZipHIVLUC2.

In pZipHIVLUC3 and 3a, the 3' splice site is deleted facilitating expression of all the HIV-LTR derived transcripts, since there is no splicing out of the sequences. These two plasmids also do not express the neo$^R$ gene, and thus selecting tester cells transfected by these plasmids is conveniently carried out by cotransfection with PSV2NEO.

### c. Preparation of pZipΔHIVLUC

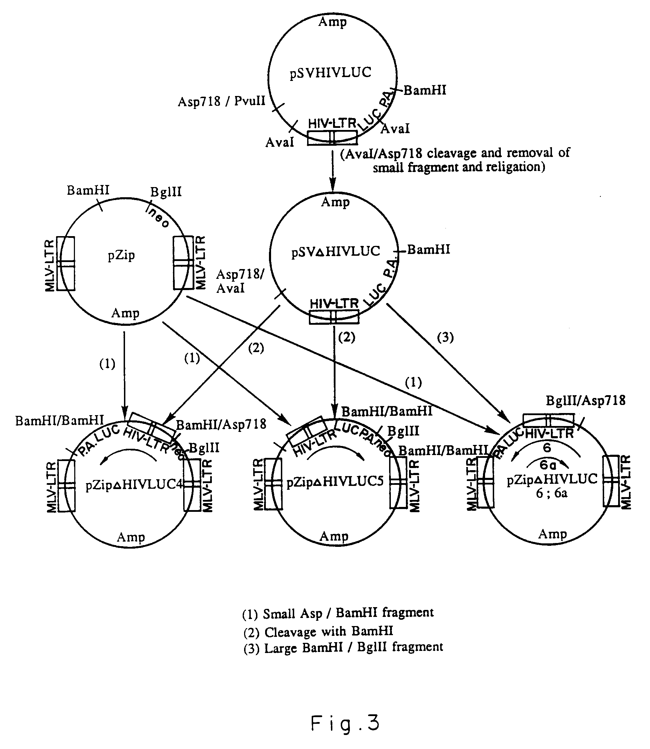

The expression of HIV is regulated by both positive and negative regulating proteins. It has been found (5), that the negative regulatory proteins interact at the 5' end of the LTR-DNA sequence upstream of the Aval cleavage site. Accordingly, similar constructs to the pZipHIVLUC series but in which the sequence of the HIV-LTR upstream of the AVaI site had been deleted, were prepared. The deletion and cloning which is schematically shown in Fig. 3 was carried out as follows:

Plasmid pSVHIVLUC was cleaved with Aval, which cleaves once at the HIV-LTR and once in the *luc* gene. Additionally, this plasmid was also cleaved with ASP718, which cleaves once in the reconstructed ASP718 site, resulting from the fusion between pVUII and ASP718 in pSVLUC12. The DNA fragment located between the ASP718 and the Aval cleavage site was removed. The two remaining DNA fragments were ligated together, the incompatible ASP718 and Aval cohesive ends were filled in using DNA polymerase Klenow fragment and ligated again. Following screening by restriction enzyme analysis, clones harbouring plasmids with the AVaI fragment inserted in the right orientation were selected.

The so-produced plasmid, pSVΔHIVLUC, was used in order to prepare the pZipΔHIVLUC4, 5, 6 and 6a. The strategy of the preparation of the plasmids was similar to that of the pZipHIVLUC plasmids. Briefly, the preparation was carried out as follows:

The ligation of the filled in Aval site in pSVHIVLUC to filled in Asp718 restriction site reconstructs the Asp718 cleavage site. The plasmid was thus cleaved with Asp718 and BamHI restriction enzymes and the so-produced ASP718-BamHI DNA fragment which contains the 160 bp deleted HIV-LTR, the *luc* gene and the SV40 poly A signal and site, was isolated. This fragment was ligated into either the BamHI cleavage site of pZip in both the sense and antisense orientation relative to MLV-LTR directed transcription, thus obtaining pZipΔHIVLUC4 and pZipΔHIVLUC5, respectively, or ligated between the BamHI and the BglII cleavage sites in the antisense or sense orientation relative to MLV transcription thus obtaining plasmids pZipΔHIVLUC6 and pZipΔHIVLUC6a, respectively.

As known (6) the PolyA signal of the cloned firefly luciferase is inefficient. By isolating the HIV-LTR/*luc*/DNA fragment from pSVLUC12, the PolyA signal and the PolyA site were added at the 3' end of the *luc* gene and since these 3' processing signals are highly efficient it facilitates efficient generation of mRNA.

In the pZipHIVLUC1, 1, 3 and 3a and pZipΔHIVLUC4, 5, 6 and 6a, the HIV-LTR promoter occlusion is ensured by the 5'-MLV-LTR and thus the background expression of luciferase is much lower than in the plasmid pALUCAT. However, out of these 8 plasmids, those in which the HIV-LTR/*luc*/Poly A was inserted in the antisense orientation, namely plasmids pZipHIVLUC1 and 3, and pZipΔHIVLUC4 and 6, are advantageous over the others. In the latter plasmids, the MLV directed transcription which may bring about production of MLV genomic RNA is truncated by processing at the HIV-LTR processing signal.

Plasmids prepared in accordance with the present invention or used in the preparation of these plasmids are listed in the following Table 1:

## Table 1

### LIST OF PLASMIDS

| Plasmid | Source | Relevant Properties |
|---|---|---|
| pSVLUC-G | J.Collins, Germany | SV40 promoter fused to *luc* gene |
| pRSVtat | J.Collins, Germany | HIV *TAT* gene fused to RSV-LTR |
| pACAT | BTG, A. Panet | *cat* gene fused to HIV-LTR |
| pBR322 | Bolivar, F. et al. 1977 (**2**) | $Amp^R$ gene |
| pBRLUC | This work | See text |
| pALUCCAT | This work | See text |
| $pSV_2CAT$ | Gorman et al. 1982 (**3**) | |
| $PSV_2NEO$ | Southern et al. 1982 (**7**) | |
| pSVHIVLUC | This work | See text |
| pSVΔHIVLUC | This work | See text |
| pZIP | Cepko, C.L. et al. 1984 (**4**) | |

Table 1 (continued)

**LIST OF PLASMIDS**

| Plasmid | Source | Relevant Properties |
|---|---|---|
| pZiPHIVLUC-1 | This work | HIV-LTRLUC insert at the BamHI site in opposite orientation to MLV transcription |
| pZiPHIVLUC-2 | This work | Same as pZiPHIVLUC-1, but the HIV-LTRLUC are inserted in the same orientation as MLV transcription |
| pZiPHIVLUC-3 | This work | HIV-LTRLUC inserted in the opposite orientation to the MLV transcription between BglII-BamHI |
| pZiPHIVLUC-3a | This work | Same as pZiPHIVLUC-3, but the HIV-LTRLUC inserted in the same orientation as MLV transcription |
| pZiPΔHIVLUC-4 | This work | Same as pZiPHIVLUC-1, but the HIV-LTR is deleted 160 bp in the 5' end |
| pZiPΔHIVLUC-5 | This work | Same as pZiPHIVLUC-2, but the HIV-LTR is deleted 160 bp in the 5' end |
| pZiPΔHIVLUC-6 | This work | Same as pZiPHIVLUC-3, but the HIV-LTR is deleted 160 bp in the 5' end |
| pZiPΔHIVLUC-6A | This work | Same as pZiPHIVLUC-3a, but the HIV-LTR is deleted 160 bp in the 5' end |

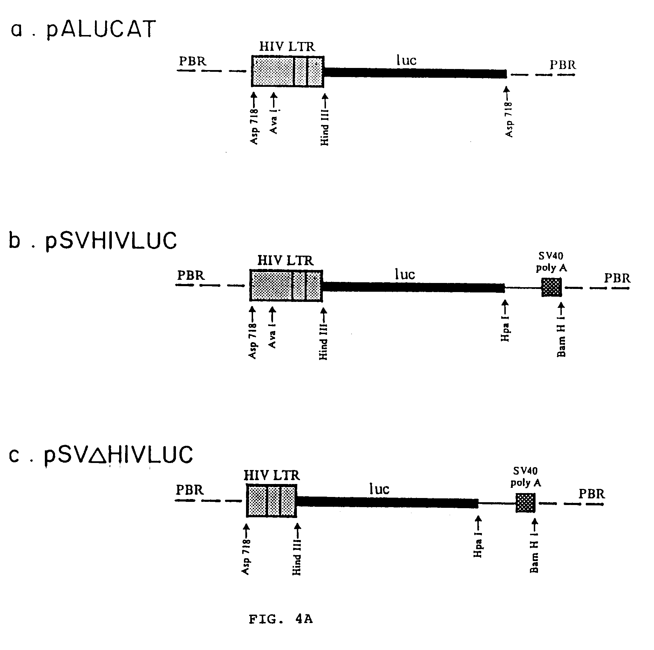

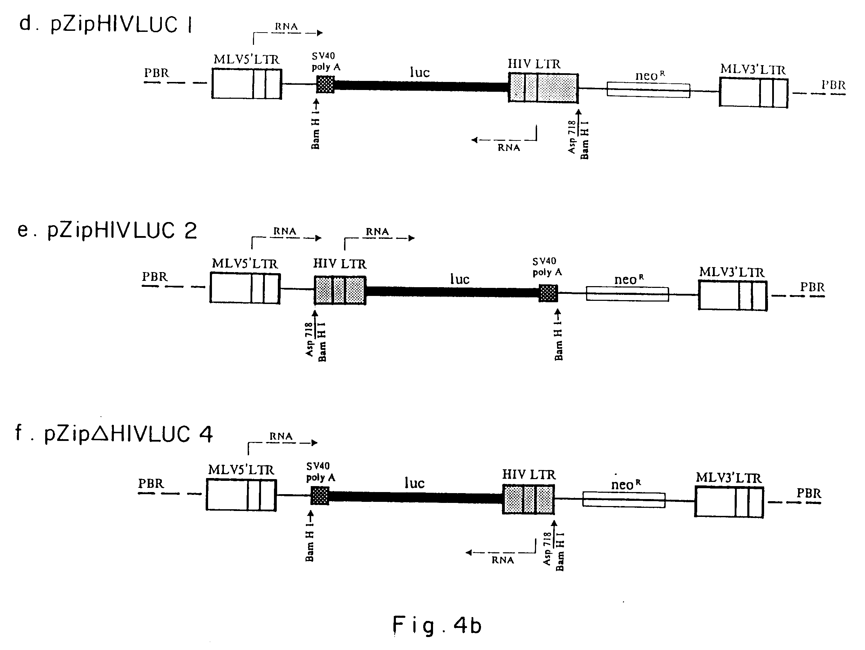

The HIV-LTR/luc region of some of the plasmids used in the following experiments is shown in Fig. 4.

**CELL LINES**

Human 293 cell line was stably transfected with pALUCAT and the gene copy number was amplified by methatroxate selection (see **8** for experimental details). These transfected cells were then infected by a CD4 carrying amphotropic retrovirus vector made in PA317 cell line following transfection with $T_4$ expressing vector (**9, 6**) and double clones were selected by neo[R] phenotype. The presence of the CD4 receptor was verified by anti-CD4 antibodies. The presence of the *luc* gene was verified by light measurements.

In a similar manner 293 cells were also transfected with pZipHIVLUC1-3a and pZipΔHIVLUC4-6a plasmids.

**MONITORING EXPRESSION OF LUCIFERASE BY LIGHT PRODUCTION**

In the following, some experiments are described in which the expression of luciferase in cells has been determined by monitoring light production. The procedures for monitoring light production were as follows:

Reference Procedure : Detection of light production in tester cell lysate

After incubation of the tester cells in accordance with the various experimental procedures, the cells were harvested, rinsed with PBS and extracts thereof were made by adding a lysis buffer (100 $\mu$l per $10^6$ cells), consisting of 1% Triton X-100, 1% BSA, 15% glycerol, 1mM DTT, 8mM $MgCl_2$, 1mM EDTA and 25mM Tris acetate pH 7.8. 10 $\mu$l of the extracts were then mixed with 250 $\mu$l of the lysis buffer, 4 $\mu$l of 40 mM ATP and then placed within a scintillation vial in a Packard liquid scintillation counter adjusted for chemiluminiscence measurements. 80 $\mu$l of 2 mM luciferin solution (Boehringer) was then added and the degree of light production in counts per minute (C.P.M.) was measured.

Inventive Procedure : Detection of light production in whole, growing tester cells.

Light emission from whole living tester cells was measured by a light detection camera (LDC) device. The LDC device consisted of a polaroid film cassette fitted with a sliding shutter and with a light-tight black box having a cover which allowed free gas flow but no light entry.

The tester cell cultures, in dishes, microtiter plates or chamber slides were fitted into holders which kept the bottom of the dish, plates or slides in direct contact with the polaroid film. Luciferin at a final concentration of 0.3mM was then added to the growth medium and the LDC was placed in a $CO_2$ incubator and the shutter was opened for desired intervals.

## EXPERIMENTAL RESULTS

### Example 1:

293 cells were transiently transfected with either the plasmid pSVΔHIVLUC or pZipΔHIVLUC4 (5$\mu$g DNA/dish) or transiently cotransfected by one of these two plasmids together with pRSV*TAT*. Light expression was monitored either on cell extracts by the method of the Reference Procedure, or on whole cells by the method of the Inventive Procedure.

The results obtained by measuring light emission in cell extracts is shown in the following Table 2:

Table 2

| Plasmid | Plasmid Alone | Cotransfection with pRSVtat | Increase after cotransfection (folds) |
|---|---|---|---|
| pSVΔHIVLUC | 2-7 x $10^{4*}$ | 5 x $10^6$ | x 100 |
| pZipΔHIVLUC4 | 3-3.5 x $10^{3*}$ | 1 x $10^7$ | x 3,000 |

*Range difference in various experiments

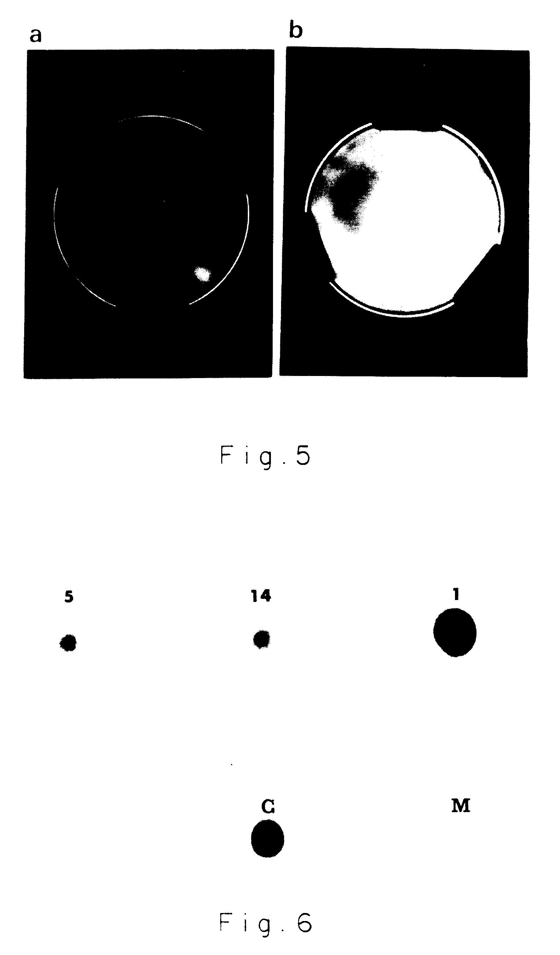

A basal light production in the two transfected cells as measured on whole, growing cells in accordance with the Inventive Procedure is shown in Fig. 5. As can be seen, the result of light production as measured by the Inventive Procedure correlates very well with those obtained by the Reference Procedure.

Furthermore as can be seen, the basal light production in pZipΔHIVLUC4 transfected cells was lower than that of the pSVΔHIVLUC transfected cells and, further, the increase in light production after cotransfection is much more pronounced in the former. These results thus clearly demonstrate the advantage of using a plasmid such as pZipΔHIVLUC4 in which the HIV-LTR promoter is occluded by the 5'MLV-LTR, over the use of a plasmid such as pSVΔHIVLUC in which no such occlusion occurs.

### Example 2:

293 cells were cotransfected with pRSV*TAT* and with pSV2NEO at a molar ratio of 10:1. The neomycin resistant clones were selected by the use of the neomycin analog, G418 (Sigma Co., U.S.A). In order to identify the *TAT* expressing clones, total RNA was obtained from each of the tester's G418 resistant clones, purified by the guanidium isothiocyanite-lithium chloride method (**10**). Equal amount of RNA was then spotted on a nitrocellulose filter and hybridized to the pRSVtat plasmid (**11**). The results from three such clones, which were arbitrarily designated as 293/*TAT*1, 293/*TAT*5 and 293/*TAT*14, is shown in Fig. 6, wherein the upper row presents results from the dot-hybridization of clones 293/*TAT*1, 293/*TAT*5 and 293/*TAT*14, the lower centre dot (C) represents positive control-hybridization with a spotted pRSVtat and the lower right corner (M) contained a dot of a negative control of RNA isolated from mock transfected 293 cells.

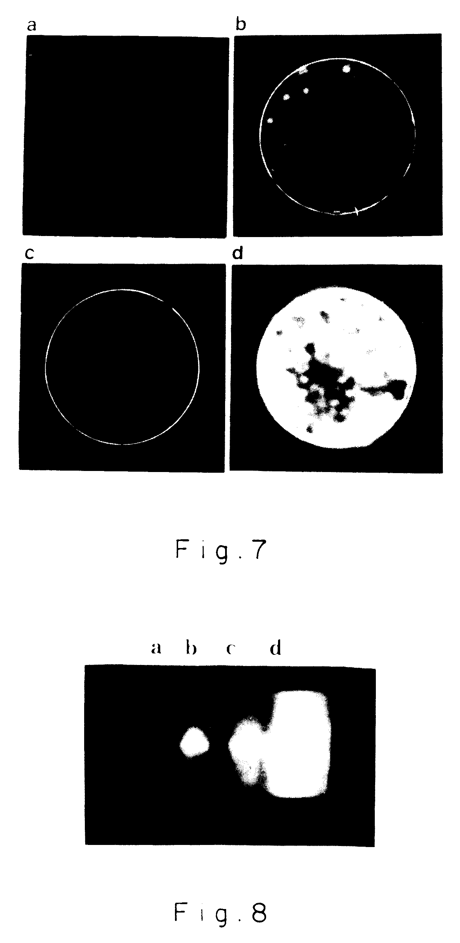

$10^5$ cells from two clones, *TAT*1 and *TAT*14 were then transfected with lug of pZipΔHIVLUC4 DNA. 24 hours following transfection, luciferin was added and light emission was monitored in the living cell in accordance with the method of Inventive Procedure. The results are shown in Fig. 7, in which a and c and b and d are the clones 293/*TAT*14 and 293/*TAT*1, respectively, a and b - 5 hours of exposure, c and d - 17 hours of exposure.

It can be clearly seen that the light emission result remarkably correlates to the amount of specific *TAT*-RNA detected by the dot-hybridization. No light emission was detected in 293/*TAT*1 cells not transfected with pZipΔHIVLUC4 (results not shown). Microscopic observations of the culture plates indicated that the light spots corresponded to aggregates of two to ten cells. This shows that the method of light detection in accordance with the Inventive Procedure may reveal *luc* activity of only one or a few cells.

## Example 3:

293 cells were transiently transfected with pZipΔHIVLUC4 and grown in a four chamber slide, 5 x $10^4$ cell/chamber. Equal aliquots of extracts of $10^5$ cells of each of the clones 293/*TAT*1, 293/*TAT*5 and 293/*TAT*14 (see Example 2) and of a control clone, 293/pRSV, transfected with the pRSV plasmid (similar to the pRSV*TAT* but not containing the *TAT* gene), were added each into one chamber and light emission was monitored in accordance with the Inventive Procedure. The light emission after exposure to extracts from the clone 293/PRSV, 293/*TAT*5, 293/*TAT*14 and 293/*TAT*1 is shown in Fig. 8a, b, c and d, respectively. As can be seen when comparing the results to those of Fig. 6, the light emission after transactivation correlates remarkably with the degree of expression of the *TAT* gene in the extracted cells.

## Example 4:

293 cells were co-transfected with pZipΔHIVLUC4 and with pSV2NEO. Independent G418 resistant clones harbouring the pZipΔHIVLUC4 plasmid were identified by light emission when grown in a multi-vale titration plate in the presence of luciferin, by the Inventive Procedure. A clone designated 293/*luc*34, which exhibited low light emission in the absence of *TAT*, was chosen for further experimentation.

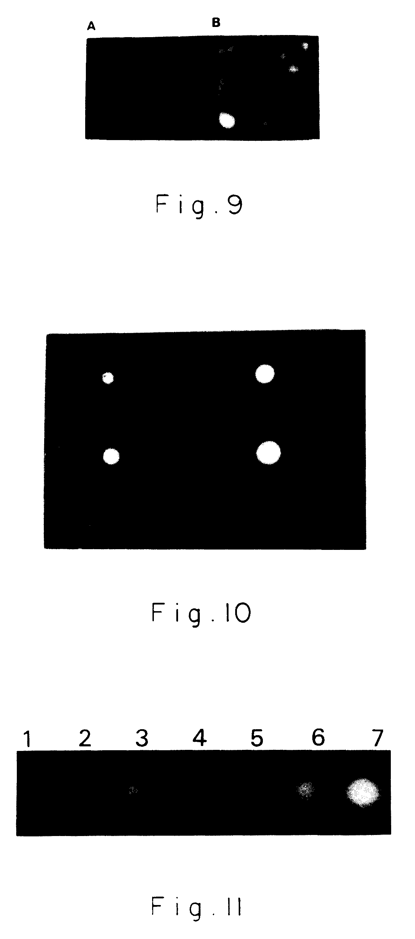

The 293/*luc*34 cells were incubated in a double chamber slide containing an equal number of cells in each chamber. Extracts from 293 cells transfected with the pRSVtat plasmid were added to one chamber, and extracts of normal, non-transfected, 293 cells were added to the other. 2 hours later, luciferin was added and light emission was determined in accordance with the Inventive Procedure. The results are presented in Fig. 9, which clearly demonstrates a considerable increase in light emission when the 293/*luc*34 tester cells were incubated in the presence of the cell extracts from pRSVtat transfected 293 cells.

It should be noted that the time of exposure after addition of luciferin depends to a great extent on the density of the tester cells and may thus be reduced considerably by their concentration. When the cells were pelleted by centrifugation in transparent test tubes, the exposure time necessary in order to record light emission by the Inventive Procedure was reduced to just a few minutes. The results of light emission from four 293/*luc*34 cell preparations concentrated in this manner, after 2 mins. of exposure, is shown in Fig. 10.

Thus, the monitoring of light production by whole, living tester cells, in accordance with the Inventive Procedure, provides an easy and quick method for the identification of the presence of *TAT* in a medium.

## Example 5:

$10^4$ 293/*luc*34 cells were inoculated into each of 7 wells and then 20μl of extract, of $10^6$ HUT78 cells (**12**) was added to one of the wells, and tenfold dilutions of equal volumes were added to each of five of the other six wells (down to $10^{-5}$ dilution). In one well, a non-infected cell extract was added as a control. Light emission was detected by the Inventive Procedure and the results are shown in Fig. 11.

As can be seen, light was emitted from all the test wells while no light emission was seen in the control well (the left well). These results also demonstrate the ability to detect very minute quantities of the *TAT* protein.

## Example 6:

Lymphocytes from blood samples of HIV sera positive and random blood donors were separated by standard density gradient centrifugation. About $10^4$ - $10^6$ cells were lysed by freezing and thawing and then the lysate was diluted ten-thousandfold and 20μl were added to wells containing $10^4$ 293/*luc*34 cells. Light emission was tested by the Inventive Procedure.

While no light emission was detected when normal blood samples were tested, all extracts from HIV sera positive blood caused light emission from the 293/*luc* cells.

## REFERENCES

1. Gold et al., (1988). Anal. Biochem. 175, 5-73.
2. Bolivar et al., (1977). Gene 2, 95-13.
3. Gorman et al., (1982). Mol. Cell Biol., 2, 1044-1051.
4. Cepko et al., (1984). Cell, 37, 1053-1062.
5. Garcia et al., (1987). EMBO, 6, 3761.
6. De Wet et al., (1987). Mol. Cell. Biol., 7, 725-737.
7. Southern et al., (1982). J. Mol. Appl. Genet., 1, 341-377.
8. Simonsen et al., (1983). Proc. Natl. Acad. Sci. U.S.A., 80, 2495-2499.
9. Maddon et al., (1986). Cell, 47, 333-348.
10. Chirguin et al., (1979). Biochemistry 18, 5294-5299.
11. Maniatis et al., (1982). A laboratory manual, Cold Spring Harbor Laboratory, Cold Spring Harbor, New York, U.S.A.
12. Gazdar et al., (1980). Blood 58, 409-417.

## Claims

1. A method for detecting the presence of an inducer substance in a cell comprising:
   a) providing tester cells prepared by
      i. constructing an expression vector comprising a promoter inducible by said inducer substance and a a *luc* gene being under the expression control of said promoter, said expression vector being capable of expressing the *luc* gene at a low level in the absence of said inducer substance and at a high level in the presence thereof,and
      ii. chosing cells, capable of absorbing luciferin from a surrounding medium and of a high level gene expression, and transfecting these cells with said expression vector;
   b) culturing the tester cells under conditions in which expression of the *luc* gene will result in light generation; and
   c) monitoring light generation by appropriate means, light generation above a certain level indicating the presence of said inducer substance in said cell.

2. The method according to claim 1, wherein the cells contain one or more genes encoding pleotropic inducers.

3. method according to Claim 1 or 2, wherein the cells are derived from the 293 cell line.

4. The method according to any one of Claims 1 to 3 for the detection of the presence of viable viral particles in a tested sample, wherein said cells are capable of being infected by the virus and said inducer substance is a substance encoded by the viral genome.

5. The method according to any one of Claims 1 to 3, for the detection of the presence of an agent in a medium, said cells being capable of absorbing said agent from a surrounding medium and said inducer substance being said agent or a substance obtained after enzymatic modification thereof.

6. The method according to any one of claims 1 to 3 for the detection in a medium of a ligand, wherein said cell comprises a receptor for said ligand on its external surface and an associated machinery for producing a second messenger, and said induces substance being said second messenger.

7. A tester cell which can generate a signal in the presence therein of an inducer substance being a cell capable of absorbing luciferase from a surrounding medium and of high level gene expression, said cell transfected with an expression vector comprising a promoter inducible by said inducer substance and a *luc* gene being under the expression control of said promoter.

8. The cell according to claim 7 comprising one or more genes encoding a pleotropic inducer.

9. The cell according to claim 8 derived from the 293 cell line.

10. The cell according to any one of claims 7 to 9 capable of absorbing said inducer substance or agent which is modified into said inducer substance by enzymes in the cell, from the surrounding medium.

11. The cell according to any one of claims 7 to 9 comprising a receptor capable of binding a ligand on its external membrane surface, binding of the ligand to said receptor causing the production in the cell of a second messenger, and said inducer substance being said second messenger.

12. The cell according to any one of claims 7 to 9 being capable of infection by a virus and said inducer substance is a substance encoded by the genome of said virus.

13. A process for preparing a cell of any one of claims 7 to 12 which can generate a signal in the presence therein of an inducer substance, comprising choosing cells which are capable of absorbing luciferin from a surrounding medium and of high level gene expression, transfecting these cells with an expression vector which comprises a promoter inducible by said inducer substance and a *luc* gene being under the expression control of said promoter, said expression vector capable of expressing the *luc* gene at a low level in the absence of said inducer substance and at a high level in the presence thereof.

14. An expression vector useful for transfecting cells in order to prepare tester cells which can generate a signal in the presence therein of an inducer substance, comprising a promoter being inducible by said inducer substance and a *luc* gene under expression control of said promoter, said expression vector capable of expressing the *luc* gene at a low level in the absence of said inducer substance and at a high level in the presence thereof.

15. The expression vector according to claim 14 comprising also a heterologous polyadenylating site and signal.

16. The expression vector according to claim 15, wherein said polyadenylating site and signal is that of SV40.

17. The expression vector according to any one of claims 14 to 16 comprising a test promoter being inducible by said inducer substance and an occluding promoter which is inducible by another inducer substance and is more active than the test promoter in the absence of the inducer substances for both promoters, the test promoter being downstream of said occluding promoter, there being no transcription termination signal between the two promoters.

18. The expression vector according to claim 17 wherein the direction of transcription induced by the test promoter is in an antisense orientation to that induced by the occluding promoter.

19. A kit for carrying out the method of any one of claims 1 to 7.

20. A system for carrying out the method of any one of claims 1 to 7.

21. The system according to claim 20 comprising a light-tight, gas permeable box mounted on a photographic film cassette, and comprising holder means for holding a cell-containing carrier in contact with the film in said cassette.

F i g . I

Fig.2

(1) Small Asp718 / BamHI fragment
(2) Cleavage with BamHI
(3) Large BamHI / BglII fragment

Fig.3

a . pALUCAT

b . pSVHIVLUC

c . pSVΔHIVLUC

FIG. 4A

d. pZipHIVLUC I

e. pZipHIVLUC 2

f. pZipΔHIVLUC 4

Fig.4b

EP 0 496 027 A1

Fig. 5

Fig. 6

a       b

c       d

Fig.7

a   b   c   d

Fig.8

Fig.9

Fig.10

Fig.11

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GENE, vol. 88, 16th April 1990, pages 197-205, Elsevier Science Publishers B.V. (Biomedical Division); O. SCHWARTZ et al.: "A microtransfection method using the luciferase-encoding reporter gene for the assay of human immunodeficiency virus LTR promoter activity" <br> * Whole document * <br>--- | 1,4,5,7,10,12,21 | C 12 Q 1/66 <br> C 12 N 15/85 <br> G 01 N 33/50 <br> C 12 Q 1/04 |
| X | WO-A-9 010 710 (DELTA BIOTECHNOLOGY LTD) <br> * Abstract; claims * <br>--- | 1,5,7,10,13,14 | |
| A | WO-A-8 801 646 (ALLELIX INC.) <br> * Claims * <br>--- | 1-21 | |
| A | TRENDS IN BIOTECHNOLOGY, vol. 6, no. 1, January 1988, pages 23-27, Elsevier Publications, Cambridge, GB; A.T. SCHAUER: "Visualizing gene expression with luciferase fusions" <br>----- | | TECHNICAL FIELDS SEARCHED (Int. Cl.5) <br><br> C 12 Q |

The present search report has been drawn up for all claims

| Place of search | Date of completion of the search | Examiner |
|---|---|---|
| THE HAGUE | 20-12-1991 | MOLINA GALAN E. |

EPO FORM 1503 03.82 (P0401)